# EUROPEAN PATENT APPLICATION

(11) **EP 3 521 436 A1**
(43) Date of publication of application: **07.08.2019**
(21) Application number: 18180095.4
(22) Date of filing: 27.06.2018
(51) Int. Cl.: C12N 15/82, A01H 1/02, A01H 1/06

(54) **COMPLEX BREEDING IN PLANTS**

(71) Applicant: VIB vzw, 9052 Gent (BE); Universiteit Gent, 9000 Gent (BE)
(72) Inventor: INZÉ, Dirk, 9310 Moorsel - Aalst (BE)

(57) **Abstract**

The present invention provides compositions and methods employing multiple guide RNAs and the Cas endonuclease system in plants for genome modification of target sequences in the genome of a plant or plant cell, for selecting plants for a desired multigenic trait, for gene editing, and for generating altered complex traits in plants.

## Description

### Field of the invention

The present application relates to the field of plant breeding, specifically to the field of molecular biology, more particularly to methods for introducing complex genetic traits in the genome of a plant or plant cell.

### Introduction to the invention

Crop productivity is determined by the highly complex interaction of numerous intrinsic genetic factors with the ever fluctuating environment. This genotype by environment (GxE) interaction is of pivotal importance in the selection of improved crops that are optimally adapted for a given environment. In some cases, such as disease resistance, the number of underlying genes responsible for the trait are limited but in many cases such as organ growth and tolerance to abiotic stress numerous genes contribute to a given favorable trait. A deeper understanding of the genetic basis of the GxE interaction not only enables the further use of marker-assisted breeding to follow superior alleles in breeding programs but also provides information that can be used for genetic modification of crops by CRISPR/Cas9 mediated genome editing. The latter technology allows for the very precise introduction of allelic variance of genes in crops. In crop breeding programs, phenotypes (e.g. related to seed yield) are collected from many different genotypes and from multi-year/multi-location field trials. By integrating the phenotypes with genetic maps of the genotypes, genomic regions can be identified that are associated with the improved trait. Using this approach, many quantitative agronomic traits were found to be determined by numerous small effect loci, the genomic regions of which are known as 'quantitative trait loci' or QTLs. Such QTLs generally are searched for in segregating mapping populations obtained from recombinant inbred lines (RILs) of two-or more parents. A more recent variant of this approach is genome-wide association study (GWAS), in which numerous genome-wide DNA markers are assayed in many diverse genotypes in order to identify loci that are associated with the desired trait. Furthermore, the combination of many phenotypic traits and the availability of a high number of genomic marker or even the entire genome sequence can be used for genomic selection to increase the predictability of the value of new breeding material. Although, these marker-assisted breeding technologies have a major impact on the accuracy and speed of crop breeding, in many case the genes underlying the QTLs are not-known. The last four decades, there has been a tremendous progress in understanding the molecular basis of many different plant processes. The use of model organisms such as *Arabidopsis* and rice has been driving these increments in knowledge. Similarly, tremendous amount of research delivered insights in the molecular pathways steering seed development, root growth and branching, plant architecture, tolerance to severe stress, cold tolerance and many more agronomic traits. All this information enforced the idea that by altering the expression of these genes plant growth and possibly crop yield could be improved. Indeed, many reports mainly resulting from *Arabidopsis* and rice research show that by modifying the expression of individual genes, positive effect of yield-relevant traits could be obtained. In our research we have identified >60 *Arabidopsis* genes, denominated intrinsic yield genes, that when overexpressed or down-regulated increase leaf size and in many cases also the size of other organs, including seeds. Furthermore, some of these genes, such as *PLASTOCHRON1,* encoding a CYTOCHROME P450 78A, also improves maize growth and when tested in hybrids resulted in a >10% increase in seed yield in multi-year, multi-location field trials (Sun et al., 2017). Similarly, numerous genes that can be used to improve seed yield and seed size in rice were identified by the academic community (for a review Li and Li, 2016). Furthermore, large number of genes, including genes studied by us, were shown to improve tolerance to abiotic stress in particular drought. Major Agro-biotech companies such as Monsanto (in a collaboration with BASF), Syngenta, Corteva (formerly known as Dupont-Pioneer/Dow Agrochemicals), Biogemma, e.a. initiated in the beginning of this century large scale programs to test for the effect of many selected genes on agronomic traits in their crops of interest, mainly in maize and rice. The conclusion of these studies was that although positive effects were often observed in greenhouse and even field trials the observed effects were too small and were too much dependent on the genotype and the environment to justify further investments in pursuing this brute-force screening approach. To illustrate the very high attrition rate of translating basic knowledge on yield-related parameters towards crop improvement we searched the Web of Knowledge ISI database and found over 5000 papers dealing with molecular aspects of drought tolerance. Despite this overwhelming number of studies until today only one genetically modified maize line is on the market with improve drought characteristics. This maize line overexpresses a *Bacillus subtilis CspB* gene encoding a RNA binding protein of which the mode of action when expressed in plants is poorly understood (Castiglioni et al, 2008).

Why is it so challenging to translate basic insights in molecular networks and genes into improved crops? Although answering this question is difficult and to some extend speculative, not unexpected, it appears that the strong genotype by environment interaction observed in breeding also affects the translatability of molecular insights. In breeding, the phenomenon of expressivity is well-known. Expressivity measures the extent to which a given genotype is expressed at the phenotypic level. The concept of expressivity is best explained by the fact that genes and their products often work in complicated networks with many different levels of regulation. This is certainly the case for processes, such as growth, that need to incorporate a panoply of endogenous, genetically determined signals as well as environmental cues. Single perturbations of networks often have a limited effect as other components of the network take over. However, in many cases the combination of perturbations of a network make phenotypes much more visible. This concept is clearly observed in breeding in which yield is determined by many small effect genes that need to work in concert to obtain maximal effects. Similarly, we previously observed that pairwise combinations of 13 Arabidopsis genes that each on their own enhanced leaf size when ectopically expressed or mutated, led in >80% of the combinations to additive or synergistic effects on leaf size (Vanhaeren et al., 2014). A triple gene-stack further enhanced the size of leaves, flowers, seeds and roots (Vanhaeren et al., 2016). Also in maize we observed, albeit with yet fewer genes that pairwise combination of growth enhancing genes revealed additive effects (Sun et al., 2017 for the PLA1 X GA20-ox combination; unpublished results for three other combinations). A major challenge is to find which combination of genes to modify. Despite the spectacular advances made by systems biology and integrating large data sets, biology is so complex that predicting which combination of genes would provide the best effects remains virtually impossible. Understanding the mode of action might be the best way+ forward to estimate the combinability of genes (Vanhaeren et al., 2014). Furthermore, even when dealing with a relative small number of genes, testing all possible pairwise gene combinations remain cumbersome and resource intensive. The latter becomes even more of a problem when triple or higher order gene combinations need to be tested which is likely needed to achieve stable double digit yield increases. Nevertheless, the potential is enormous. For example, we previously showed that a triple combination of three different mutants of growth promoting genes (*DA1; EOD1* and *SAMBA*) increased the size of leaves, flowers, seeds and even roots of Arabidopsis in a spectacular manner (Vanhaeren et al., 2016).

There is clearly a need for a high throughput method to be able to screen for (and identify) specific gene combinations, which when these genes are modified, lead to agronomically valuable traits in crops.

### Summary of the invention

In the present invention we satisfy this need by bridging the gap between classical genetics (involving the entire genome) and genetic engineering of single genes by combining multiplex CRIPSR-mediated genome editing with breeding for selecting favorable phenotypes. This groundbreaking approach will not only lead to improved crops but will simultaneously deliver unprecedented knowledge on the molecular networks of complex genetic traits such as for example organ growth and abiotic stress tolerance. We have designated our new method BREEDIT, a word which marks the fusion of breeding and editing.

The present invention provides compositions and methods employing multiple single guide RNAs /Cas endonuclease system in plants for genome modification of selected target sequences in the genome of a plant or plant cell, for selecting plants, for gene editing, and for modifying polynucleotides of interest present in the genome of a plant. The methods and compositions employ a multiple guide RNAs/Cas endonuclease system to provide for an effective system for modifying or altering target sites and nucleotides of interest within the genome of a plant, plant cell or seed. Breeding methods and methods for selecting plants utilizing a two component multiple single RNA guides and Cas endonuclease system are also disclosed. Also provided are nucleic acid constructs, plants, plant cells, explants, seeds and grain having the multiple single guide RNA/Cas endonuclease system. Compositions and methods are also provided employing a multiple single guide polynucleotide/Cas endonuclease system for genome modification of target sequences in the genome of a cell or organism, for gene editing, and for altering specific genes of interest in the genome of a plant cell or plant. The methods and compositions employ a multiple single guide polynucleotide RNAs/Cas endonuclease system to provide for an effective system for modifying or altering target sites and editing nucleotide sequences of interest within the genome of a cell, wherein the multiple single guide polynucleotides can comprise of a RNA sequence, a DNA sequence, or a DNA-RNA combination sequence.

Thus in a first aspect the invention provides a method to produce a crop having an altered complex trait locus comprising at least two altered target gene sequences in a genomic region of interest, the method comprising i) providing a recombinant DNA construct capable of expressing a multiplicity of sgRNAs to a first parent plant of a crop which comprises a chimeric Cas endonuclease gene expressing a functional Cas endonuclease wherein said guide RNAs and Cas endonuclease are capable of forming a complex that enables the Cas endonuclease to introduce double-stranded breaks in at least 2 genes present in said crop, ii) repeating step i) in a second parent plant of said crop with a multiplicity of sgRNAs having specificity for a different set of genes than in step i), iii) optionally repeating step ii) in more parent plants of said crop with a multiplicity of gRNAs having a specificity for different set of genes than in the previous steps, iv) randomly crossing homozygous lines of the parent plants of said crop obtained in steps i), ii) and iii) in an open pollination crossing scheme, v) selecting a progeny plant having a desired complex trait.

In a specific aspect the at least 2 targeted gene alterations are present in the promoter and/or the coding sequence and/or the terminator of said genes.

In yet another specific aspect the at least two targeted gene alterations are present on different chromosomes in the crop.

In yet another specific aspect the at least two targeted gene alterations in the method are gene disruptions.

In yet another specific aspect the at least two targeted gene alterations in the method are a combination of a gene disruption and a gene alteration.

In yet another aspect the crop in the methods described herein before is a monocot or a dicot.

In a specific aspect the crop in the methods described herein before is selected from the group consisting of maize, rice, sorghum, rye, barley, wheat, millet, oats, sugarcane, turfgrass, switchgrass, soybean, canola, alfalfa, sunflower, cotton, tobacco, peanut, potato, tobacco, safflower, poplar or eucalyptus.

In yet another aspect the complex trait in the method is yield or abiotic stress tolerance or tolerance to pathogen infection.

In yet another aspect the Cas endonuclease gene used in the methods is a plant optimized Cas9 endonuclease.

In yet another specific aspect the sgRNAs in the methods are provided to the plant directly by particle bombardment.

In yet another specific aspect the sgRNAs in the methods are provided to the plant by particle bombardment or *Agrobacterium* transformation of a recombinant DNA construct comprising the corresponding sgRNAs operably linked to a plant U6 polymerase III promoter.

In yet another specific aspect a plant or seed is obtained by any one of the methods described herein before.

In some of these aspects, the Cas endonuclease gene is a plant optimized Cas9 endonuclease.

In some of these aspects, the Cas endonuclease gene is operably linked to a SV40 nuclear targeting signal upstream of the Cas codon region and a VirD2 nuclear localization signal downstream of the Cas codon region.

### Figures

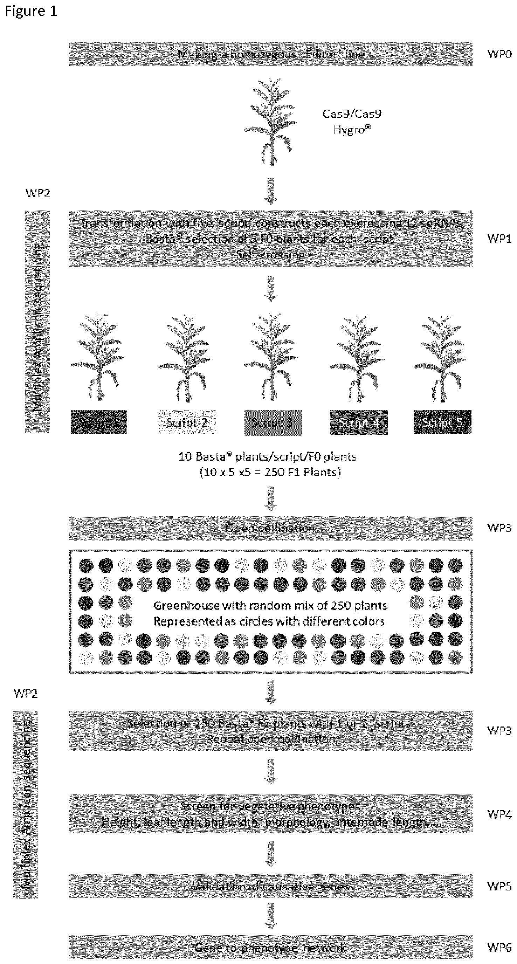

Figure 1: BREEDIT consists of several steps. First (see example 1) a maize line homozygous for a single locus insertion of *Cas9,* further referred to as the 'editor' line is generated. Next (example 2), the 'editor' line is transformed with constructs (further referred to as 'script' constructs) each containing different sets of sgRNAs directed against target genes of interest. For clarity, the figure shows 5 'script' constructs each having 12 different sgRNAs. Next, the different so-called 'script' lines (Script1, Script2, Script3...) are self-crossed and the F1 progeny used for open pollination in green house conditions (examples 4 and 5). Such open pollination allows for combining all possible combination of two 'scripts' constructs in the resulting F2 population. Next, these F2 plants (containing 1 or 2 different 'script' constructs) are grown together in a greenhouse (WP4), once more allowing open pollination, resulting in an F3 population that also will be used for screening of desired phenotypes (see example 6). Causative genes will be validated using genome editing (see example 7). Throughout the BREEDIT scheme, efficiency of genome editing will be monitored using Multiplex amplicon sequencing (see example 3).

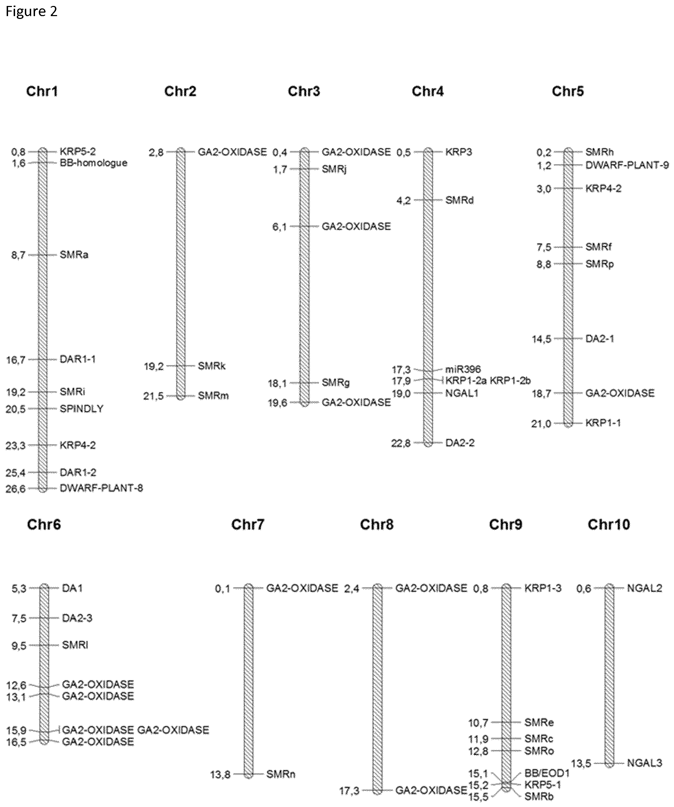

Figure 2: The position of the some selected 'intrinsic yield' genes on the maize chromosomes that can be used in the context of the BREEDIT method is shown

### Detailed description of the invention

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., current Protocols in Molecular Biology (Supplement 100), John Wiley & Sons, New York (2012), for definitions and terms of the art. The definitions provided herein should not be construed to have a scope less than understood by a person of ordinary skill in the art.

The present disclosure includes compositions and methods for genome modification of at least two target sequences in the genome of a plant or plant cell, for selecting plants, for gene editing, and for altering at least two genes of interest in the genome of a plant. The methods employ multiple guide RNAs/Cas endonuclease system, wherein the Cas endonuclease is guided by the single guide RNAs to recognize and introduce a double strand break at specific target sites into the genome of a cell. The multiple single guide RNAs /Cas endonuclease system provides for an effective system for modifying target sites within the genome of a plant, plant cell or seed. Breeding methods utilizing a two component multiple single guide RNAs/Cas endonuclease system are also disclosed. Compositions and methods are also provided for editing at least two nucleotide sequence in the genome of a plant cell. The nucleotide sequences to be edited (the nucleotide sequence of interest) can be located within or outside a target site that is recognized by a Cas endonuclease.

The basic idea of BREEDIT is to use a crop (herein further exemplified with maize), each maize expressing Cas9 and a different set of single guide RNAs as starting material for plant breeding. The single guide RNAs are directed to a set of chosen genes (herein exemplified by intrinsic yield genes and/or drought tolerance genes) of which the combinatorial inactivation yields favorable phenotypes (see Figure 1 for the general BREEDIT method).

Specifically the strategy of BREEDIT (exemplified for maize) comprises the following steps:
1. Generate a maize B104 line homozygous for single locus, active insertion of *Cas9,* further referred to as the 'editor' line.
2. Transform the 'editor' line with constructs (further referred to as 'script' constructs each containing different sets of sgRNAs (single guide RNAs) directed against target genes of interest ('intrinsic yield') genes. As a result, various primary transgenic plants are obtained each expressing different sets of sgRNAs.
3. The different so-called 'script' lines (Script1, Script2, Script3,...) are self-crossed and the F1 progeny used for open pollination in green house conditions. Such open pollination will allow for generating all possible combination of two 'scripts' constructs in the resulting F2 population.
4. Next, these F2 plants will be grown together in a greenhouse, once more allowing open pollination, resulting in an F3 population that also will be used for screening of desired phenotypes. At this stage also new F1 or F2 plants with different script constructs can be added in the open pollination set-up.
5. Plants with a desired phenotype will be submitted to multiplex amplicon sequencing to identify mutation in the different target genes used to make the single guide RNAs for the various 'scripts'.
6. Confirm the causative nature of the identified genes by designing and testing a 'script' construct in which only the genes that are found mutated in the plants with the desired phenotype are present.

BREEDIT aims at developing a strategy to combine genome editing with breeding and to enhance the finding of complex gene combinations that affect important crop traits. The general methodology is exemplified herein by using the maize inbred line B104 because its ease of transformation. The BREEDIT approach can be used to improve germplasm that is used for the development of commercial hybrids and recent developments in the capacity to regenerate plants (e.g.BABYBOOM/WUSHEL technology, (Lowe et al, 2018)) will considerably facilitate this. The skilled artisan will appreciate that the method can be applied to many different crops including soybean, sorghum, rice and other crops further exemplified herein.

Importantly, in 2018 USDA announced that it will not regulate plants that could otherwise have been developed through traditional breeding techniques including plants that contain deletions, single base pair substitutions, insertion from compatible plant relatives and complete null segregants of a genetically engineered plant. The BREEDIT approach will be a major step forward in integrating basic knowledge on genes with plant breeding. This technology has the potential to become a game changer in improving crop yield. Such increased crop yield is not only important for feeding the ever growing demands but it also may represent an enormous economic value. For example, we calculated the economic impact of a 10% increase for corn yield in the US based on the following three facts (USDA): i) in 2016 38 Mio ha of corn were grown; ii) the average yield in 2016 was 10.98 tons/ha; iii) US corn commodity price is 134.8 USD/tons. The potential value of 10% yield increase for the US corn by selecting new gene combination would be 10.98 tons/ha x 38 Mio ha x 13.48 USD/tons or 5.6 billion USD annually. In Europe, where a non-GMO solution would be marketable, 57 M ton of maize is grown, with Romania, Hungary, Italy and France as main producers. It is expected that simple gene edits will not be seen as GMO products and therefor the potential for improving crop yield in corn in Europe is tremendous.

Therefore in a first embodiment the invention provides a method to produce a crop having a complex trait locus comprising at least two altered target gene sequences in a genomic region of interest, the method comprising i) providing a recombinant DNA construct capable of expressing a multiplicity of sgRNAs to a first parent plant of a crop which comprises a chimeric Cas endonuclease gene expressing a functional Cas endonuclease wherein said sgRNAs and Cas endonuclease are capable of forming a complex that enables the Cas endonuclease to introduce double-stranded breaks in at least 2 genes present in said crop, ii) repeating step i) in a second parent plant of said crop with a multiplicity of sgRNAs having specificity for a different set of genes than in step i), iii) optionally repeating step ii) in more parent plants of said crop with a multiplicity of sgRNAs having a specificity for different set of genes than in the previous steps, iv) randomly crossing homozygous lines of the parent plants of said crop obtained in steps i), ii) and iii) in an open pollination crossing scheme, v) selecting a progeny plant having a desired complex trait.

The CRISPR-Cas technology originates from type II CRISPR-Cas systems, which provide bacteria with adaptive immunity to viruses and plasmids. The CRISPR system has been repurposed using Cas9 as the pioneer endonuclease to induce RNA-programmable gene knockout and editing (Barrangou & Doudna 2016 Nat Biotechnol 34:933-941; Wang et al 2016 Ann Rev Biochem 85:227-264; Wright et al 2016 Cell 164:29-44). The CRISPR associated protein Cas9 is an endonuclease that uses a guide sequence within an RNA duplex, tracrRNA:crRNA, to form base pairs with DNA target sequences, enabling Cas9 to introduce a site-specific double-strand break in the DNA. The dual tracrRNA:crRNA was then engineered as a single guide RNA (sgRNA) that retains two critical features: the 20-nucleotide sequence at the 5' end of the sgRNA (protospacer) that determines the DNA target site by Watson-Crick base pairing, and the double-stranded structure at the 3' side of the guide sequence that binds to Cas9 (Jinek et al 2012 Science 337:816-821). This created a simple two-component system in which changes to the guide sequence (20 nucleotides in the native RNA) of the sgRNA can be used to program CRISPR-Cas9 to target any DNA sequence of interest as long as it is adjacent to a protospacer adjacent motif (PAM) (Jinek et al 2012 Science 337:816-821). Thus, the Cas9-sgRNA complex will bind any target genomic sequence with a PAM, but Cas9 only cleaves the target genomic sequence if sufficient homology exists between the sgRNA spacer and target genomic sequence. The PAM sequence is a short sequence motif adjacent to the CRISPR RNA-targeted sequence that is critical for initial DNA binding (Doudna & Charpentier 2014 Science 346:12580961-12580969). Indeed, in the absence of the PAM, even target sequences fully complementary to the sgRNA sequence are not recognized by the endonuclease. The end result of Cas9-mediated DNA cleavage is a double strand break (DSB) within the target sequence, at a cleavage site that is about 3-4 nucleotides upstream of the PAM sequence. The simplicity of CRISPR-Cas9 programming, together with a unique DNA cleaving mechanism, the capacity for multiplexed target recognition, and the existence of many natural type II CRISPR-Cas system variants, has enabled remarkable developments using this cost-effective and easy-to-use technology to precisely and efficiently target, edit, modify, regulate, and mark genomic loci of a wide array of cells and organisms.
Once the power of the CRISPR-Cas technology for genome editing purposed was recognized, many other Cas endonucleases were identified and functionally analyzed. One example is Cpf1 (Cas12a). The genome editing potential of Cpf1 has been confirmed in human cells for several members of the current Cpf1-family, including *Francisella novicida* Cpf1 (FnCpf1), *Acidaminococcus* sp. BV3L6 Cpf1 (AsCpf1), and *Lachnospiraceae* bacterium ND2006 Cpf1 (LbCpf1) (Zetsche et al 2015 Cell 163:759-771).

It will be appreciated that the terms Cas enzyme, CRISPR enzyme, CRISPR protein, CRISPR endonuclease, Cas endonuclease, CRISPR effector protein, Cas protein and CRISPR Cas are generally used interchangeably and at all points of reference herein refer by analogy to Cas endonucleases further described in this application. A classification of CRISPR-Cas systems was proposed in WO2016205711A1. Class 1 uses several Cas proteins together with the CRISPR RNAs to build a functional endonuclease. Class 2 CRISPR systems use a single Cas protein with a crRNA. In particular embodiments, the CRISPR-Cas system described herein is a Class 2 system. CRISPR systems that may be used in the practice of the invention vary greatly. CRISPR systems can be a type I, a type II, a type II, a type IV, a type V or a type VI system. Non-limiting examples of suitable CRISPR proteins include Cas1, Cas1B, Cas2, Cas3, Cas4, Cas5, Cas5e (or CasD), Cas6, Cas6e, Cas6f, Cas7, Cas8,Cas8a1, Cas8a2, Cas8b, Cas8c, Cas9, Cas10, Cas12 (or Cpf1), Cas13a (or C2c2), Cas1O, Cas1 Od, CasF, CasG, CasH, Csy1, Csy2, Csy3, CseI (or CasA), Cse2 (or CasB), Cse3 (or CasE), Cse4 (or CasC), Csc1, C2c1, Csc2, C2c3, Csa5, Csn2, Csm2, Csm3, Csm4, Csm5, Csm6, Cmr1, Cmr3, Cmr4, Cmr5, Cmr6, Csb1, Csb2, Csb3, Csx1, Csx17, Csx14, Csx100, Csx16, CsaX, Csx3, Csz1, Csx15, Csf1, Csf2, Csf3, Csf4, and Cul966 or homologues thereof.
In some embodiments, the CRISPR protein is derived from a class II type V CRISPR system, even more particularly a type V-A. Cpf1 has been recently identified as a Class 2, Type V CRISPR-Cas systems containing a 1,300 amino acid protein.
The Cas protein for this and other functions set out herein can be from *Streptococcus pyogenes, Streptococcus thermophilus, Streptococcus* sp., *Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas* sp., *Crocosphaera watsonii, Cyanothece* sp., *Microcystis aeruginosa, Synechococcus* sp., *Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter* sp., *Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc* sp., *Arthrospira maxima, Arthrospira platensis, Arthrospira* sp., *Lyngbya* sp., *Microcoleus chthonoplastes, Oscillatoria* sp., *Petrotoga mobilis, Thermosipho africanus, Acidaminococcus* sp., *Lachnospiraceae* sp. or *Acaryochloris marina.* Genome editing is a game-changing technology and almost on a daily basis there are reports on new 'nickases'; nickases which recognize a different PAM sequence, base-editing nickases,... The skilled person will understand that many components of the BREEDIT strategy can be modified. Examples include modifications of the promoter driving Cas9; ii) the number of sgRNAs in the script constructs; iii) the use of other nickases or base-editing deaminases.
BREEDIT mostly relies on Cas9 mediated base pair deletions (see above) that often lead to gene inactivation. Mutations that lead to up-regulation (in cis) of the expression and/or activity of target genes are more difficult to produce but are not excluded from the scope of the present invention. An example of the latter are mutations that modify promoter sequences (mutation of repressor binding sites) and consequently alter the expression of the target gene. Alternatively, one might search for gene edits that remove gene sequences encoding degrons or auto-inhibitory sequences. Furthermore, one could look for in frame edits in which miRNA binding sites are mutated.

Furthermore, in BREEDIT the screening for phenotypes can be conveniently carried out on seedlings but obviously if space allows plants could be grown to maturity in green houses or phenotyped directly on (semi-)-automated platform. The latter allows for the direct assaying of more yield phenotypes such a cob length and width. A step further would even be to look for phenotypes in field grown plants using screen houses (outdoor fields which are covered with nets). Such screen houses are extensively used in China to evaluate the performance of transgenic maize plants (Prof. Jingbing Yan, personal communication).

During the last years we extensively used Cas9 mediated genome editing in maize. From the 26 single guideRNAs tested to date in maize in our research, only one guide never resulted in edited plants, making the CRISPR/Cas9 system highly efficient (>96%) to induce mutations. Of the guide RNAs that caused mutations, the majority was bi-allelic (>39%) or heterozygote (>26%), while over 6% was homozygous in the primary transformed plants. A single guide RNA can cause different types of alleles, which range from small InDels (most frequently 1 or 2 nucleotides causing a frame shift) to deletions of the entire sequence between two sgRNAs targeting the same gene (unpublished results). Furthermore, we have experimentally observed that the Cas9 remains active in generating new mutations (in the presence of the sgRNAs) in subsequent generations, when wild type alleles are still present or are introduced by a backcross.

In a specific embodiment, the Cas endonuclease gene is a Cas9 endonuclease. In another embodiment, the Cas endonuclease gene is plant, maize or soybean optimized Cas9 endonuclease In another embodiment, the Cas endonuclease gene is operably linked to a SV40 nuclear targeting signal upstream of the Cas codon region and a bipartite VirD2 nuclear localization signal (see Tinland et al. (1992) Proc. Natl. Acad. Sci. USA 89:7442-6) downstream of the Cas codon region.

The terms "functional fragment", "fragment that is functionally equivalent" and "functionally equivalent fragment" are used interchangeably herein. These terms refer to a portion or subsequence of the Cas endonuclease sequence of the present disclosure in which the ability to create a double-strand break is retained.

In one embodiment, the Cas endonuclease gene is a plant codon optimized streptococcus pyogenes Cas9 gene that can recognize any genomic sequence of the form N(12-30)NGG can in principle be targeted.

In one embodiment, the Cas endonuclease is introduced directly into a cell by any method known in the art, for example, but not limited to transient introduction methods, transfection and/or topical application.

As used herein, the term "single guide RNA" (sgRNA) relates to a synthetic fusion of two RNA molecules, a crRNA (CRISPR RNA) comprising a variable targeting domain, and a tracrRNA. In one embodiment, the guide RNA comprises a variable targeting domain of 12 to 30 nucleotide sequences and a RNA fragment that can interact with a Cas endonuclease.

In one embodiment, the sgRNA and Cas endonuclease are capable of forming a complex that enables the Cas endonuclease to introduce a double strand break at a chosen DNA target site in the genome of a plant.

In one embodiment of the disclosure the variable target domain is 12, 13, 14, 15, 16, 17, 18, 19, 20, 21, 22, 23, 24, 25, 26, 27, 28, 29 or 30 nucleotides in length.

In one embodiment of the disclosure, the sgRNA comprises a cRNA (or cRNA fragment) and a tracrRNA (or tracrRNA fragment) of the type II CRISPR/Cas system that can form a complex with a type II Cas endonuclease, wherein said single guide RNA/Cas endonuclease complex can direct the Cas endonuclease to a plant genomic target site, enabling the Cas endonuclease to introduce a double strand break into the genomic target site.

In one embodiment the sgRNA can be introduced into a plant or plant cell directly using any method known in the art such as, but not limited to, particle bombardment or topical applications.

In another embodiment the sg RNA can be introduced indirectly by introducing a recombinant DNA molecule comprising the corresponding guide DNA sequence operably linked to a plant specific promoter that is capable of transcribing the sgRNA in said plant cell. The term "corresponding sgDNA" includes a DNA molecule that is identical to the RNA molecule but has a "T" substituted for each "U" of the RNA molecule.

In some embodiments, the guide RNA is introduced via particle bombardment or *Agrobacterium* transformation of a recombinant DNA construct comprising the corresponding guide DNA operably linked to a plant U6 polymerase III promoter.

In one embodiment, the RNA that guides the RNA/Cas9 endonuclease complex, is a duplexed RNA comprising a duplex crRNA-tracrRNA. One advantage of using a single guide RNA versus a duplexed crRNA-tracrRNA is that only one expression cassette needs to be made to express the fused single RNA.

The terms "target site", "target sequence", "target DNA", "target locus", "genomic target site", "genomic target sequence", "gene alteration" and "genomic target locus" are used interchangeably herein and refer to a polynucleotide sequence in the genome (including choloroplastic and mitochondrial DNA) of a plant cell at which a double-strand break is induced in the plant cell genome by a Cas endonuclease. The target site can be an endogenous site in the plant genome, or alternatively, the target site can be heterologous to the plant and thereby not be naturally occurring in the genome, or the target site can be found in a heterologous genomic location compared to where it occurs in nature. As used herein, terms "endogenous target sequence" and "native target sequence" are used interchangeable herein to refer to a target sequence that is endogenous or native to the genome of a plant and is at the endogenous or native position of that target sequence in the genome of the plant.

An "artificial target site" or "artificial target sequence" are used interchangeably herein and refer to a target sequence that has been introduced into the genome of a plant. Such an artificial target sequence can be identical in sequence to an endogenous or native target sequence in the genome of a plant but be located in a different position (i.e., a non-endogenous or non-native position) in the genome of a plant.

An "altered target site", "altered target sequence", "modified target site", "modified target sequence", "gene alteration" are used interchangeably herein and refer to a target sequence as disclosed herein that comprises at least one alteration when compared to non-altered target sequence. Such "alterations" include, for example:
(i) replacement of at least one nucleotide, (ii) a deletion of at least one nucleotide, (iii) an insertion of at least one nucleotide, or (iv) any combination of (i) - (iii).

Thus "a gene alteration" in the context of the present invention lead to the expression of a functional gene but the expression can be enhanced or reduced (hence the term 'altered') while a "gene disruption" means a non-functional gene which is a gene which is not expressed anymore and is essentially a knockout of a gene.

The length of the target site can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other cases, the incisions could be staggered to produce single-stranded overhangs, also called "sticky ends", which can be either 5' overhangs, or 3' overhangs.

As used herein, a "genomic region" is a segment of a chromosome in the genome of a plant cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5-70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases.

Error-prone DNA repair mechanisms can produce mutations at double-strand break sites. The NonHomologous-End-Joining (NHEJ) pathways are the most common repair mechanism to bring the broken ends together (Bleuyard et al., (2006) DNA Repair 5:1 -12). The structural integrity of chromosomes is typically preserved by the repair, but deletions, insertions, or other rearrangements are possible. The two ends of one double-strand break are the most prevalent substrates of NHEJ (Kirik et al., (2000) EMBO J 19:5562-6), however if two different double-strand breaks occur, the free ends from different breaks can be ligated and result in chromosomal deletions (Siebert and Puchta, (2002) Plant Cell 14:1 121-31), or chromosomal translocations between different chromosomes (Pacher et al., (2007) Genetics 175:21 -9). Once a double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1 -12). The structural integrity of chromosomes is typically preserved by the repair, but deletions, insertions, or other rearrangements are possible (Siebert and Puchta, (2002) Plant Cell 14:1121 -31 ; Pacher et al., (2007) Genetics 175:21 -9). Alternatively, the double-strand break can be repaired by homologous recombination between homologous DNA sequences. Once the sequence around the double-strand break is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52).

Methods for identifying at least one plant cell comprising in its genome a polynucleotide of interest integrated at the target site.

Further provided are methods for identifying at least one plant cell, comprising in its genome, an altered polynucleotide of interest at the target site. A variety of methods are available for identifying those plant cells with alterations in the genome at or near to the target site without using a screenable marker phenotype. Such methods can be viewed as directly analyzing a target sequence to detect any change in the target sequence, including but not limited to PCR methods, sequencing methods, nuclease digestion, Southern blots, and any combination thereof. The method also comprises recovering a plant from the plant cell comprising one or more alterations at specific target sites in the genome.

Complex traits of interest include, but are not limited to, abiotic and biotic stress tolerance or plant traits such as yield, grain quality, nutrient content, starch quality and quantity, nitrogen fixation and/or utilization, fatty acids, and oil content and/or composition. More specific traits of interest include, but are not limited to, resistance to abiotic stress, such as drought, nitrogen, temperature, salinity, toxic metals or trace elements, or those conferring resistance to toxins such as pesticides and herbicides, or to biotic stress, such as attacks by fungi, viruses, bacteria, insects, and nematodes, and development of diseases associated with these organisms. Also it is understood that agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch.

It is to be understood that although particular embodiments, specific configurations as well as materials and/or molecules, have been discussed herein for cells and methods according to the present invention, various changes or modifications in form and detail may be made without departing from the scope and spirit of this invention. The following examples are provided to better illustrate particular embodiments, and they should not be considered limiting the application. The application is limited only by the claims.

### Examples

### 1. Generation of a corn 'editor' line

In the present example a B104 maize line homozygous for a construct expressing *Cas9* ('editor' line) is constructed. We have observed that CRISPR/Cas9 mediated genome editing in maize is very efficient. Indeed, the from the 26 guide RNAs (gRNAs) tested in maize, only one single guide never resulted in edited plants, making the CRISPR/Cas9 system highly efficient (>96%) to induce mutations. Of the single guide RNAs that caused mutations, the majority was bi-allelic (>39%) or heterozygote (>26%), while over 6% was homozygous in the primary transformed plants. A single guide RNA can cause different types of alleles, which range from small InDels (most frequently 1 or 2 nucleotides causing a frame shift) to deletions of the entire sequence between two gRNAs targeting the same gene. Furthermore, we have experimentally observed that the Cas9 remains active in generating new mutations (in the presence of the gRNAs) in subsequent generations, when wild type alleles are still present or are introduced by a backcross.

We construct a maize B104 line that constitutively expresses *Cas9* (with a for maize optimized codon usage) under the control of the constitutive UBL promoter. B104 is chosen as inbred to work on as it is relatively easy to transform with *Agrobacterium* (Coussens et al.,2012) and because we have generated a large amount of data on the molecular networks that drive growth and tolerance to mild drought stress (large sets of unpublished results). Hygromycine is used as a selectable marker and primary transgenic shoots are tested for the presence of a single copy T-DNA by quantitative PCR analysis (). To eliminate somaclonal variation, several of these transgenic F0 shoots are crossed with B104 and hygromycine resistant offspring are selfed again and homozygous F2 plants are selected by testing for the presence of two T-DNA copies. Initially three of these plants, homozygous for *Cas9,* plants, called 'editor' are up-scaled and immature embryos of this plant are used for transformation with the various 'script' constructs (see example 2). When the molecular analysis (see example) shows that the CRISPR induced mutation frequency is comparable in all three lines, we will continue with one 'editor' line.

### 2. Design and transformation of 'script' constructs

In the present example we design a number of different constructs (called 'scripts') that each express 12 different single guide RNAs (sgRNA). We use five 'script' constructs wherein each script construct contains 12 sgRNAs and each sgRNA targets a different gene. Expression of each individual sgRNA is driven by either the rice OsU3 promoter or the wheat TaU3 promoter. In case the target genes are part of highly conserved gene families, sgRNAs against highly homologous regions are designed that address multiple if not all members of the gene families. These 'multi-gene' sgRNAs are expected to make mutations without bias in members of the conserved gene families and as such mutations in random combinations of these genes can be found. The sgRNAs are cloned in a T-DNA vector containing a BASTA resistance (BASTA^{R}) gene but no *Cas9* (for an outline see Figure 2). In short, vectors have already been made that can be used with the golden gate cloning method to construct 12 sgRNAs in three intermediate vectors with each gRNA under the control of the rice OsU3 promoter or wheat TaU3 promoter. Next the three intermediate vectors are inserted in a binary vector with the BASTA^{R} selectable marker using gateway cloning. These vectors are sequence verified and introduced in *Agrobacterium tumefaciens* (EHA101). It is expected that, when transformed to the 'editor' plants, the sgRNAs in the 'script' constructs will generate various heterozygous, homozygous mutations as well as bi-allelic mutations (different mutations in each of the alleles of a target gene). To have an idea of the frequency of such mutations, multiplex amplicon sequencing (MTA-Seq, see example 3) is used on five independent BASTA^{R} primary transgenic plants to analyze the frequency of mutations in the 12 target sites, respectively. The MTA-seq method allows for simultaneous monitoring the occurrence of mutations in all selected genes by using a bar-coded PCR amplification. With the latter method many different plants can be analyzed by one sequencing reaction (for further details see example 3). This 'mutation profiling' is used to follow the efficiency of gene editing not only in the primary transgenics but also in subsequent crosses. Initially, five independent primary transformed plants in which the sgRNAs have worked to generate mutations are self-crossed. This procedure is repeated with five different 'script' constructs containing each 12 different sgRNAs targeting different sets of genes. In principle crossing primary transgenic plants is also possible but the major hurdle is that plants suffer from the tissue culture and do not mature at the same time. The nature of the genes to be analyzed is determined by the biological question. In the present invention gene combinations are sought for which determine the size of plant organs. To the end, we BREEDIT is used to identify gene combinations that improve growth of maize organs. As genome editing in its simplest form frequently produces frame shift mutations (see above), BREEDIT will make use of genes for which it is known or expected (mainly from research in Arabidopsis and/or rice) that the inactivation leads to enhanced plant (or organ) growth. These genes are further referred to as intrinsic yield genes (IYG). We use 60 sgRNA (12 different sgRNAs in each of the 5 'script' constructs) and for specific gene families, it is possible to design sgRNAs that target multiple members of these gene families. We have selected, albeit not limiting, the maize genes listed below to be further used in BREEDIT.
- The two DELLA-encoding genes (*DWARF PLANT8* and *DWARF PLANT9*).
- 13 maize *GA2-OXIDASEs* that mediate the inactivation of bioactive GAs.
- SPINDLY mediates *O*-fucosyl transferase of DELLA and activates its growth-repressing activity. In Arabidopsis, *spy* mutants display elevated responses to gibberellin (Zentella et al.,2017).
- The proteases DA1, DAR1-1 and DAR1-2 that when activated cleave and inactivate proteins that positively regulate cell cycle progression. Inactivation of DA1 was shown to enhance leaf and seed size in Arabidopsis (Li et al., 200). DA1 related proteins are regulated at the protein level by ubiquitinilation that activates the protease activity. This ubiquitinilation is mediated by the E3 ligases BIG BROTHER (EOD1/BB) and BB-homologues DA2-1, DA2-2 and DA2-3. Inactivation of EOD1/BB enhances organ growth in Arabidopsis.
- The gene encoding the miR396 that targets 16 of the 18 maize GROWTH REGULATING FACTORS (GRFs). The GRFs positively regulate maize growth and the inactivation of miR396 enhances organ growth in Arabidopsis.
- The transcription factors NGAL1, NGAL2 and NGAL3. In Arabidopsis, the expression of a *PLA1-*related gene, an homolog of *KLUH,* is under control of two redundantly acting repressor proteins, NGAL2 and NGAL3, both belonging to the NGATHA-like protein family. The simultaneous inactivation of both transcription factors was shown to increase seed size in Arabidopsis (Zhang et al, 2015). It is expected that inactivation of either of the three maize *NGAL* genes, or more likely two or three genes, will enhance *PLA1* expression and hence biomass and grain yield.
- Inhibitors of CYCLIN DEPENDENT KINASES (CDKs), kinase with a pivotal role in driving cell cycle progression. Plants have two classes of CDK inhibitory proteins KIP-RELATED PROTEINS (KRPs) and SIAMESE/SIAMESE RELATED (SIM/SMR). Inactivation of multiple KRPs result in increased organ size in Arabidopsis (Cheng et al, 2013). Maize has 8 *KRPs* genes (Godinez-Palma et al., 2018) and encodes 16 SIAMESE and SIAMESE RELATED proteins. All 8 KRP genes can be targeted by 5 sgRNAs while for the *SIAMESE* and *SIAMESE RELATED* genes can be inactivated by 9 sgRNAs.
- This list can be extended in order to target as many genes as possibly, using 60 sgRNAs (to be part of five script constructs).

The position of the selected 'intrinsic yield' genes is shown in Figure 2.

The various 'script' constructs are introduced in the B104 maize 'editor' line using *Agrobacterium* mediated transformation of immature embryo's. Transgenic plants are selected for resistance to the herbicide BASTA. To ensure that as much as possible independent events (with independent alleles) are selected, only plants (five in total) derived from calli originating from independent embryos are further analysed. To maximize the chances that combinations of mutations are found by crossing various 'script' plants (see example 4) that the sgRNAs within one 'script' construct are preferably targeting genes located on different chromosomes, or at least far enough apart on one chromosome to allow for efficient recombination. Maize has 10 chromosomes and as shown in Figure 2 target genes are well distributed over the different chromosomes. In order to assure that the different 'script' work well multiplex amplicon sequencing (example 3) is used to monitor the occurrence of mutations in the primary transgenic plants.

### 3. Monitoring the efficiency of genome editing throughout BREEDIT

The aim of the present example is to use multiplex amplicon sequencing to assay for the efficiency of genome editing in the different generations of maize plants (F0, F1, F2 and F3 plants). The frequency of mutations is followed in a subset of plants. To this end, multiplex amplicon sequencing of all target genes is used. The method of choice is the recently described 'Multiplex PCR Targeted Amplicon Sequencing' (MTA-Seq) (see Onda et al., 2018) . In short, primer pairs are designed to generate amplicons of the target genes. Next a highly multiplexed PCR is conducted and the amplicons are end-repaired. Subsequently, sample specific (bar-coding) adapters are ligated, the library PCR amplified and sequenced. With this method hundreds of genes can be followed simultaneously in up to 96 individual plants. Prior to the final selection of the various sgRNAs and the construction of the 'scripts' we also test whether the selected amplicons consistently work on genomic maize DNA. We have observed that some target sites are, for unknown reasons, difficult to amplify and therefore are not useful to detect mutations.

### 4. Combination of different 'scripts'

In this example different 'script' constructs are combined with each other by using an open pollination set-up in which plants with different 'scripts' are crossed with each other. For each 'script' construct, five different primary transgenic lines (F0) showing different patterns of edits are retained and self-crossed.

As discussed above, we have observed that already in the F0 generation bi-allelic edits can frequently be found. Furthermore, we know that upon self-crossing the 'editor' remains active and continue to produce new mutations. Next, F1 plants obtained by self-crossing of the F0 plants are subjected to a BASTA^{R} leaf painting assay to ensure that the F1 plants are either heterozygous (50%) or homozygous (25%) for the 'script' construct. For each script construct 10 BASTA^{R} F1 plants are retained. Next, BASTA^{R} F1 plants harboring different 'scripts' (5 scripts X 5 plants /script X 10 BASTA^{R} F1 seedlings = 250 plants) are grown next to each other in a dedicated greenhouse and used for open pollination. At anthesis/silking, plants are daily extensively moved in the greenhouse to ensure that as much as possible genetic diversity (combining different 'scripts') is generated. This open pollination also assures that wild-type alleles are introduced. As many of the F1 plants already contain homozygous 'edited' genes, the open pollination introduces again genetic diversity. Such open pollination allows for combining all possible combinations of two 'scripts' in the resulting F2 population and in addition self-pollination results in homozygous mutants in single or multiple genes derived from one 'script'. As all F2 plants are homozygous for the cas9 'editor' also new mutations will be generated. To follow the process, selected F2 plants are subjected to a molecular analysis using multiplex amplicon sequencing (see example 3). Next, 250 BASTA^{R} F2 plants containing all different single 'scripts' and double 'script' combinations are grown again together for open pollination. This breeding step further increases the number of possible genes for which the combinatorial effect of their inactivation can be addressed. In the resulting F3 population up to 36 sgRNAs can be expressed (from the combination of three 'scripts') and the combinatorial effect of inactivation of the corresponding target genes is studied. At all stages multiplex amplicon sequencing is used to monitor the frequency of mutations (see example 3). F3 seeds are used for further phenotyping in the green house.

### 5. Phenotyping of maize plants derived from the open pollinations and identification of genes that when mutated (in combination) affect vegetative phenotypes

In this example the offspring of the open pollination (F2 and F3) is screened and combinations of mutations generated by the different 'script' constructs are determined. Seeds of the F3 open pollinations are germinated in pots and grown at high density in the greenhouse. Control B104 plants are mixed amongst the 'edited' F2 or F3 plants. Plants are examined for any distinguishable phenotype such as plant height, leaf length, internode length, leaf width, timing of leaf appearance, leaf color,... Plants are randomized on a daily basis to avoid position effects. Plants with distinguishable phenotypes are further analyzed and grown until maturity. During the further development of these selected plants we also monitor reproductive development including cob and tassel development as well a seed related parameters. Furthermore, plants with aberrant phenotypes will be molecularly characterized using multiplex amplicon sequencing (see WP2). It is expected that the plants with distinguishable phenotypes reveal mutations in combinations of genes induced by the genome editing mediated by the cas9 in the 'editor' plants and the various 'script' constructs. Using MTA-seq (see example 3) and comparing the pattern of mutations present in mutants with similar phenotypes putative causative genes are identified. The causal nature of these genes is further analyzed in example 6. We also analyze by MTA-seq lines that do not show the phenotype as this makes it possible to rule out certain genes/combinations of genes. Selected mutant plants are also self-crossed to maintain the mutations.

### 6. Validation of causative genes.

In the present example the causal nature of the genes that are putatively responsible for the phenotypes observed in example 5 are validated. The causative nature of the identified genes in example 5 are validated by designing and testing a 'script' construct in which only sgRNAs for the genes that are found mutated in the plants with the desire phenotype are present. Essentially, a similar pipeline is used as described above with the exception that the number of sgRNAs is limited to the candidate genes. Designing the 'validation script' construct is straightforward as the building blocks are already available for example 1. Depending on the number of genes to validate and chromosomal position of these genes one or two 'validation script' constructs are used. If the identified genes are, when mutated, causative for the expected phenotype, we find F2 and/or F3 plants with such phenotype. This experiment learns if all genes need to be mutated in order to observe the anticipated phenotype. Plants with the anticipated phenotype are further grown on the PHENOVISION platform (www.psb.ugent.be/phenotyping/phenovision), an automated plant phenotyping system that allows to image maize plants throughout the entire life cycle. PHENOVISION has been extensively deployed in the AMAIZE project and was fine-tuned to analyze maize growth under well-watered and mild drought conditions.

### References

1. Sun, X., et al., Altered expression of maize PLASTOCHRON1 enhances biomass and seed yield by extending cell division duration. Nat Commun, 2017. 8: p. 14752.
2. Li, N. and Y. Li, Signaling pathways of seed size control in plants. Current Opinion in Plant Biology, 2016. 33: p. 23-32.
3. Castiglioni, P., et al., Bacterial RNA Chaperones Confer Abiotic Stress Tolerance in Plants and Improved Grain Yield in Maize under Water-Limited Conditions. Plant Physiology, 2008. 147(2): p. 446-455.
4. Vanhaeren, H., et al., Combining growth-promoting genes leads to positive epistasis in Arabidopsis thaliana. eLife, 2014. 3: p. e02252.
5. Vanhaeren, H., D. Inze, and N. Gonzalez, Plant Growth Beyond Limits. Trends in Plant Science, 2016. 21(2): p. 102-109.
6. Lowe, K., et al., Rapid genotype "independent" Zea mays L. (maize) transformation via direct somatic embryogenesis. In Vitro Cellular & Developmental Biology, 2018. 54(3): p. 240-252.
7. Baute, J., et al., Correlation analysis of the transcriptome of growing leaves with mature leaf parameters in a maize RIL population. Genome Biology, 2015. 16(1): p. 168.
8. Baute, J., et al., Combined Large-Scale Phenotyping and Transcriptomics in Maize Reveals a Robust Growth Regulatory Network. Plant Physiology, 2016. 170(3): p. 1848-1867.
9. Feys, K., et al., Growth rate rather than growth duration drives growth heterosis in maize B104 hybrids. Plant, Cell & Environment, 2018. 41(2): p. 374-382.
10. Voorend, W., et al., Overexpression of GA20-OXIDASE1 impacts plant height, biomass allocation and saccharification efficiency in maize. Plant Biotechnology Journal, 2016. 14(3): p. 997-1007.
11. Nelissen, H., N. Gonzalez, and D. Inze, Leaf growth in dicots and monocots: so different yet so alike. Curr Opin Plant Biol, 2016. 33: p. 72-76.
12. Besbrugge, N., et al., GS(yellow), a Multifaceted Tag for Functional Protein Analysis in Monocot and Dicot Plants([OPEN]). Plant Physiology, 2018. 177(2): p. 447-464.
13. Zhang, Y., et al., Transcription factors SOD7/NGAL2 and DPA4/NGAL3 act redundantly to regulate seed size by directly repressing KLU expression in Arabidopsis thaliana. Plant Cell, 2015. 27(3): p. 620-32.
14. Cheng, Y., et al., Downregulation of multiple CDK inhibitor ICK/KRP genes upregulates the E2F pathway and increases cell proliferation, and organ and seed sizes in Arabidopsis. The Plant Journal, 2013. 75(4): p. 642-655.
15. Godínez-Palma, S.K., et al., Two maize Kip-related proteins differentially interact with, inhibit and are phosphorylated by cyclin D-cyclin-dependent kinase complexes. Journal of Experimental Botany, 2017. 68(7): p. 1585-1597.

## Claims

1. A method to produce a crop having an altered complex trait locus comprising at least two altered target gene sequences in a genomic region of interest, the method comprising i) providing a recombinant DNA construct capable of expressing a multiplicity of gRNAs to a first parent plant of a crop which comprises a chimeric Cas endonuclease gene expressing a functional Cas endonuclease wherein said guide RNAs and Cas endonuclease are capable of forming a complex that enables the Cas endonuclease to introduce double-stranded breaks in at least 2 genes present in said crop, ii) repeating step i) in a second parent plant of said crop with a multiplicity of gRNAs having specificity for a different set of genes than in step i), iii) optionally repeating step ii) in more parent plants of said crop with a multiplicity of gRNAs having a specificity for different set of genes than in the previous steps, iv) randomly crossing homozygous lines of the parent plants of said crop obtained in steps i), ii) and iii) in an open pollination crossing scheme, v) selecting a progeny plant having a desired complex trait.

2. A method according to claim 1 wherein said at least 2 targeted gene alterations are present in the promoter and/or the coding sequence and/or the terminator of said genes.

3. A method according to claims 1 or 2 wherein said at least two targeted gene alterations are present on different chromosomes.

4. A method according to any one of claims 1 to 3 wherein said at least two targeted gene alterations are gene disruptions.

5. A method according to any one of claims 1 to 3 wherein said at least two targeted gene alterations are a combination of a gene disruption and a gene alteration.

6. A method to produce a crop according to any one of claims 1 to 5 wherein said crop is a monocot or a dicot.

7. A method according to claim 6 wherein said crop is selected from the group consisting of maize, rice, sorghum, rye, barley, wheat, millet, oats, sugarcane, turfgrass, switchgrass, soybean, canola, alfalfa, sunflower, cotton, tobacco, peanut, potato, tobacco, safflower, poplar or eucalyptus.

8. A method according to any one of claims 1 to 7 wherein said complex trait is yield or abiotic stress tolerance.

9. A method according to any one of claims 1 to 8 wherein the Cas endonuclease gene is a plant optimized Cas9 endonuclease.

10. A method according to any one of claims 1 to 9 wherein the gRNAs are provided to the plant directly by particle bombardment.

11. A method according to any one of claims 1 to 9 wherein the gRNAs are provided to the plant by particle bombardment or *Agrobacterium* transformation of a recombinant DNA construct comprising the corresponding gRNAs operably linked to a plant U6 polymerase III promoter.

12. A plant or seed obtained by any one of the methods 1 to 11.
